# EUROPEAN PATENT SPECIFICATION

(11) **EP 3 557 244 B1**
(45) Date of publication and mention of the grant of the patent: **14.04.2021**
(21) Application number: 19150856.3
(22) Date of filing: 08.01.2019
(51) Int. Cl.: G01N 29/24, B06B 1/02

(54) **ULTRASONIC EXAMINATION DEVICE AND ULTRASONIC PROBE**
ULTRASCHALLUNTERSUCHUNGSVORRICHTUNG UND ULTRASCHALLSONDE
DISPOSITIF D'EXAMEN À ULTRASONS ET SONDE À ULTRASONS

(30) Priority: 19.04.2018 JP 2018080728
(43) Date of publication of application: 23.10.2019
(73) Proprietor: Hitachi, Ltd., Tokyo 100-8280 (JP)
(72) Inventor: TAKEZAKI, Taiichi, Chiyoda-ku,, Tokyo 100-8280 (JP); HASEGAWA, Hiroaki, Chiyoda-ku,, Tokyo 100-8280 (JP); MACHIDA, Shuntaro, Chiyoda-ku,, Tokyo 100-8280 (JP); IMAI, Ryo, Chiyoda-ku,, Tokyo 100-8280 (JP); TANAKA, Tomohiko, Chiyoda-ku,, Tokyo 100-8280 (JP)
(74) Representative: Strehl Schübel-Hopf & Partner

(56) References cited:
- US-A1- 2017 366 104
- CALIANO GIOSUE ET AL: "Biasing of Capacitive Micromachined Ultrasonic Transducers", IEEE TRANSACTIONS ON ULTRASONICS, FERROELECTRICS AND FREQUENCY CONTROL, IEEE, US, vol. 64, no. 2, 1 February 2017 (2017-02-01), pages 402-413, XP011640312, ISSN: 0885-3010, DOI: 10.1109/TUFFC.2016.2623221 [retrieved on 2017-02-02]

## Description

### Technical Field

The present invention relates to an ultrasonic examination device and an ultrasonic probe, and more particularly, to effective technology applied to an ultrasonic examination device and an ultrasonic probe which use a capacitive ultrasonic transducer manufactured by micro electro mechanical system (MEMS) technology.

### Background Art

Ultrasonic sensors are utilized in various ultrasonic examination devices such as medical ultrasonic echo diagnostic devices, nondestructive ultrasonic flaw detection devices, or the like, and for example, are used in blood vessel catheters for examining vasoocclusion or vasodilation of a living body.

Related-art ultrasonic sensors normally use oscillation of a piezoelectric substance, but, with the recent enhancement of MEMS technology, capacitive ultrasonic transducers (capacitive micro-machined ultrasonic transducer (CMUT)) using MEMS technology are developing.

The capacitive ultrasonic transducer has an oscillator formed on a semiconductor substrate and having a cavity formed between electrodes facing each other. The capacitive ultrasonic transducer generates ultrasonic waves by overlappingly applying voltages of a direct current and an alternating current to the respective electrodes, and vibrating a membrane (flexible film) in the vicinity of a resonance frequency.

Technology relating to the ultrasonic examination device described above is disclosed, for example, in patent literature 1 (JP-A-2014-136002). In patent literature 1, there is disclosed connecting a high-capacity capacitor which is charged by receiving power supply from an ultrasonic transducer to the ultrasonic transducer.

Non patent literature "Biasing of Capacitive Micromachined Ultrasonic Transducers" (CALANO GIOSUE et A1, IEEE TRANSACTIONS ON ULTRASONICS, FERROELECTRICS AND FREQUENCY CONTROL, IEEE, US, vol. 64, no. 2, 1 February 2017 (2017-02-01), pages 402-413)discloses an ultrasonic examination device comprising: a capacitive ultrasonic transducer which is formed of a first condenser which comprises a first electrode, a cavity on the first electrode, and a second electrode formed on the cavity;a second condenser which comprises a third electrode and a fourth electrode above the third electrode, and is connected to the first condenser in parallel.

### Citation List

### Patent Literature

PTL 1: JP-A-2014-136002

### Summary of Invention

### Technical Problem

In order to transmit and receive ultrasonic waves, and to obtain an image by using an ultrasonic examination device, a direct current voltage should be always applied to an ultrasonic transducer. An ultrasonic examination device using an ultrasonic transducer in a blood vessel catheter uses the direct current voltage of 200 V, for example, but it is apprehended that a current leaks into a blood vessel when a short circuit breakdown occurs, and there is a need to take secure measures against a direct current bias voltage.

To solve these problems, a method of mounting a battery in an ultrasonic probe having an ultrasonic transducer mounted therein, and supplying a direct current bias voltage to the ultrasonic transducer from the battery is suggested. In addition, a method of using, as the battery, a condenser (capacitor) formed in a semiconductor chip different from a semiconductor chip having an ultrasonic transducer mounted therein, is suggested. However, in the above-described structures, there is a great parasitic capacitance between the condenser and the ultrasonic transducer which are mounted in the different semiconductor chips, and, since an electric charge is easily discharged from the condenser, there is a difficulty in operating the ultrasonic transducer for a long time.

The scope of the invention is defined in the appended claims. The above-described objects and novel features of the present invention will become apparent based on the descriptions and the accompanying drawings of the present specification.

### Solution to Problem

A summary of what is disclosed in the present application is described briefly as follows.

An ultrasonic examination device includes a cell which is a capacitive type device, and a charging condenser, the cell and the charging condenser being mounted in one semiconductor chip and being connected with each other in parallel, and further includes a switch which switches ON/OFF of connection between the cell and a DC bias power source.

Another disclosed ultrasonic probe includes a cell which is a capacitive type device, and a charging condenser, the cell and the charging condenser being mounted in one semiconductor chip and being connected with each other in parallel, and further includes a switch which switches ON/OFF of connection between the cell and a DC bias power source.

### Advantageous Effects of Invention

Effects achieved by representative embodiments of the invention disclosed in the present application are briefly described as follows.

According to the present invention, performance of the ultrasonic examination device can be enhanced.

According to the present invention, performance of the ultrasonic probe can be enhanced.

### Brief Description of Drawings

[Fig. 1] Fig. 1 is a top view illustrating a semiconductor chip forming an ultrasonic examination device according to a first embodiment which is not forming part of the claimed scope of the present invention.
[Fig. 2] Fig. 2 is a cross-sectional view taken on line A-A of Fig. 1.
[Fig. 3] Fig. 3 is a block diagram illustrating a configuration of the ultrasonic examination device according to the first embodiment.
[Fig. 4] Fig. 4 is a cross-sectional view of the semiconductor chip forming the ultrasonic examination device in the process of manufacturing according to the first embodiment.
[Fig. 5] Fig. 5 is a cross-sectional view of the semiconductor chip in the process of manufacturing, continuing from Fig. 4.
[Fig. 6] Fig. 6 is a cross-sectional view of the semiconductor chip in the process of manufacturing, continuing from Fig. 5.
[Fig. 7] Fig. 7 is a cross-sectional view of the semiconductor chip in the process of manufacturing, continuing from Fig. 6.
[Fig. 8] Fig. 8 is a block diagram illustrating a configuration of the ultrasonic examination device according to a variation of the first embodiment, this variation forming part of the claimed scope of the present invention.
[Fig. 9] Fig. 9 is a timing chart illustrating an operation of the ultrasonic examination device according to the variation of the first embodiment.
[Fig. 10] Fig. 10 is a cross-sectional view illustrating a semiconductor chip forming an ultrasonic examination device according to a second embodiment of the present invention.
[Fig. 11] Fig. 11 is a cross-sectional view illustrating a semiconductor chip forming an ultrasonic examination device according to a third embodiment which is not forming part of the claimed scope of the present invention.
[Fig. 12] Fig. 12 is a block diagram illustrating a configuration of the ultrasonic examination device according to the third embodiment.
[Fig. 13] Fig. 13 is a cross-sectional view illustrating the semiconductor chip forming the ultrasonic examination device according to a first variation of the third embodiment.
[Fig. 14] Fig. 14 is a cross-sectional view illustrating the semiconductor chip forming the ultrasonic examination device according to a second variation of the third embodiment.
[Fig. 15] Fig. 15 is a perspective view illustrating an ultrasonic examination device according to a fourth embodiment of the present invention.
[Fig. 16] Fig. 16 is a cross-sectional view illustrating an ultrasonic probe according to a fifth embodiment of the present invention.

### Description of Embodiments

Hereinafter, embodiments of the present invention will be described in detail with reference to the drawings. Throughout the drawings for describing embodiments, the same reference numerals are used for the elements having the same functions, and a redundant explanation thereof will be omitted. In the following embodiments, explanation of the same or similar portions will not be repeated in principle except for the special case.

### (First Embodiment)

An ultrasonic examination device according to the first embodiment is an ultrasonic wave transmission and reception sensor of a capacitance detection type, which is provided with a capacitive ultrasonic transducer manufactured by using micro electro mechanical system (MEMS) technology, for example. Herein, mix-mounting, in one semiconductor chip, a condenser forming a capacitive type device cell, and a charging condenser for supplying a direct current bias voltage to the capacitive type device cell will be described.

### <Structure of the Ultrasonic Examination device of the Present embodiment>

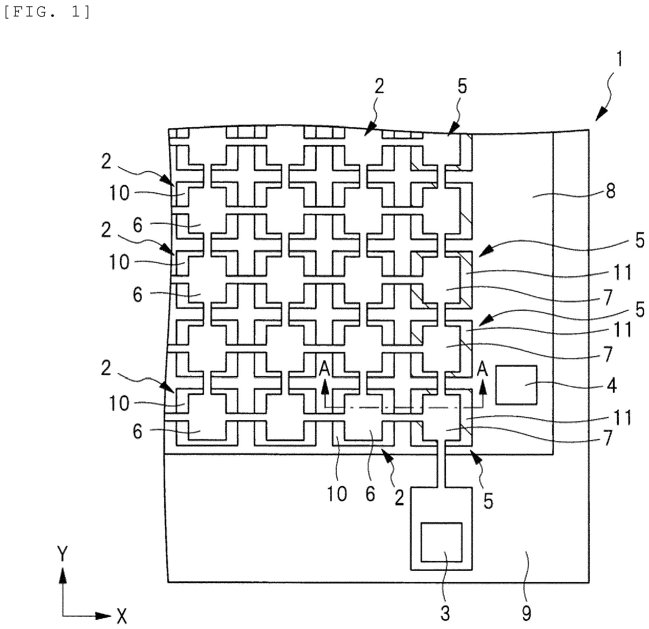

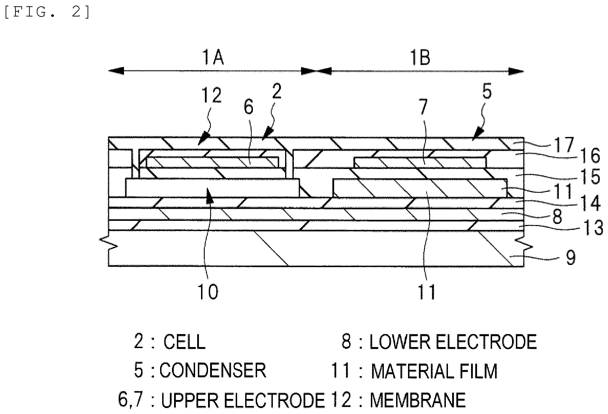

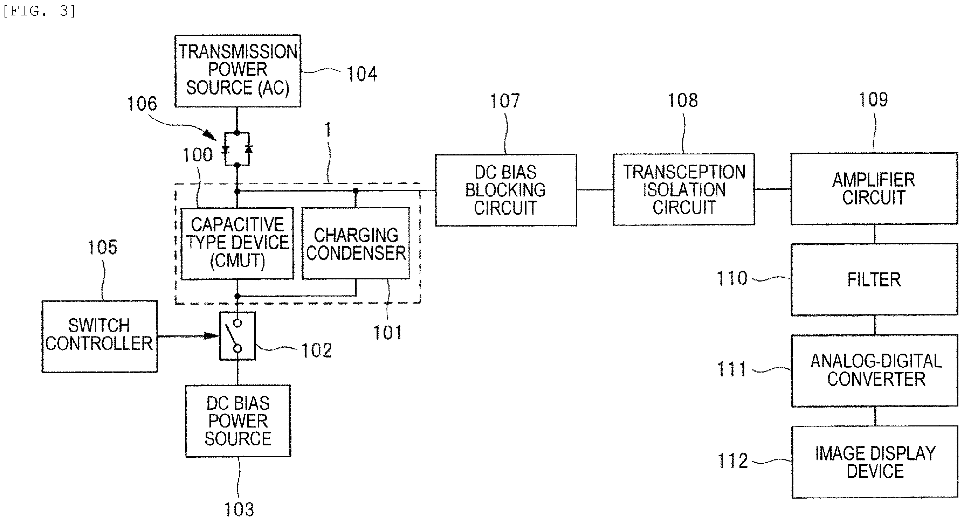

Hereinafter, a configuration of the ultrasonic examination device according to the present embodiment will be described with reference to Figs. 1 to 3. Fig. 1 is a top view illustrating the semiconductor chip forming the ultrasonic examination device according to the present embodiment. Fig. 2 is a cross-sectional view illustrating the semiconductor chip forming the ultrasonic examination device according to the present embodiment. Fig. 2 is a cross-sectional view taken on line A-A of Fig. 1. Fig. 3 is a block diagram illustrating a configuration of the ultrasonic examination device according to the present embodiment.

FIG. 1 is a top view illustrating a portion of the semiconductor chip 1 forming the ultrasonic examination device according to the present embodiment. The semiconductor chip 1 includes a main surface (an upper surface, a surface) and a rear surface (a lower surface) which are disposed at opposite sides along a thickness direction, and Fig. 1 illustrates a top view (upper surface view) of the main surface side of the semiconductor chip 1. Fig. 1 does not illustrate insulation films such as interlayer films, etc., and mainly illustrates a substrate, a lower electrode and an upper electrode which are stacked on the substrate, a gap and a film (sacrifice film) which is disposed between the upper electrode and the lower electrode, and an electrode pad.

A planar shape of the semiconductor chip 1 is, for example, an oblong, that is, a rectangle. The semiconductor chip 1 includes a substrate 9, and a plurality of cells 2 arranged in an X direction and a Y direction, a plurality of condensers 5, and bonding pads (hereinafter, referred to as pads) 3 and 4 are arranged on the main surface of the substrate 9. The X direction and the Y direction are directions which are orthogonal to each other in planar view, and both directions are directions parallel to the main surface of the semiconductor chip 1 and the main surface of the substrate 9. That is, the plurality of cells 2 are arranged in the form of a matrix. The pads 3 and 4 are terminals for inputting to and outputting from the semiconductor chip 1, and bonding wires, etc. are electrically connected to the pads 3 and 4.

The plurality of condensers 5 are arranged in the Y direction in an area adjacent to a cell array having the cells 2 arranged in the form of a matrix in planar view. In the X direction, the condensers 5 are arranged adjacent to the cells 2 of an edge of the cell array. The cell array is formed on a center portion of the semiconductor chip 1 in planar view. The plurality of condensers 5 are formed in the proximity of an edge of the semiconductor chip 1 in planar view. Herein, the case in which the condensers 5 are adjacent to the cell array in the X direction will be described, but the condensers 5 are not necessarily adjacent to the cell array, and may be arranged in any position as long as they are arranged in the semiconductor chip 1.

The cell 2 is a capacitive type device cell formed by a condenser which is a variable capacitance. In the present application, the capacitive type device provided with the plurality of cells 2 is referred to as a capacitive ultrasonic transducer (capacitive micro-machines ultrasonic transducer (CMUT)). In the present application, the capacitive ultrasonic transducer may be simply referred to as an ultrasonic transducer. Each cell 2 includes one cavity (gap) 10. A shape of the cavity 10 in planar view is, for example, an rectangle. The cavities 10 forming the plurality of cells 2 are spaced from one another. Each cell 2 includes a membrane (flexible film) 12 (see Fig. 2) formed on the cavity 10 in an area overlapping the cavity 10 in planar view. Each of the plurality of cells 2 is an oscillator of a minimum unit which is able to generate ultrasonic waves (vibrate) and to transmit the ultrasonic waves, and also, is able to receive ultrasonic waves. The oscillator forms, for example, an electrostatic type variable capacitance (variable capacitance sensor).

The cell 2 includes a lower electrode 8, the cavity 10 on the lower electrode 8, and an upper electrode 6 forming a portion of the membrane 12 on the cavity 10. That is, the lower electrode 8, the cavity 10, and the upper electrode 6 overlap one another in planar view. The plurality of cells 2 share one lower electrode 8. The lower electrode 8 is widely formed to cover almost the whole semiconductor chip 1, such that the lower electrode 8 overlaps the cell array (cell area) having the plurality of cells 2 arranged therein, and the condenser area having the plurality of condensers 5 arranged therein, respectively, in planar view.

In contrast, the condenser 5 is a capacitance element which is not a variable capacitance. That is, a capacitance of the condenser 5 is not changed and is always constant. Each of the plurality of condensers 5 is a charging condenser which is used to apply a direct current voltage to the cell 2. The condenser 5 includes the lower electrode 8 and an upper electrode 7 on the lower electrode 8. That is, the lower electrode 8 and the upper electrode 7 overlap each other in planar view. The plurality of condensers 5 share one lower electrode 8 with one another. In addition, the plurality of cells 2 and the plurality of condensers 5 share one lower electrode 8 with one another. A gap is not formed between the lower electrode 8 and the upper electrode 7, and a material film (a sacrifice film, an interelectrode film) 11 is formed therebetween. In Fig. 1, hatching is applied to the material film 11 for easy understanding of the drawing.

Among the plurality of upper electrodes 6 forming the plurality of cells 2, the upper electrodes 6 adjacent to each other in the X direction or the Y direction are connected to each other by a metal wire which is integrally formed with the upper electrode 6. In addition, the upper electrode 6 forming the cell 2, and the upper electrode 7 forming the condenser 5 are connected to each other by a metal wire formed of a metal film integrally formed with the upper electrodes 6 and 7. That is, the condenser forming the cell 2, and the condenser 5 have their respective upper electrodes 6 and 7 electrically connected to each other, and their respective lower electrodes 8 electrically connected. That is, the condenser forming the cell 2, and the condenser 5 are connected to each other in parallel. The planar shape of each of the upper electrodes 6 and 7 is a rectangle, and a width of the metal wire in a short side direction, connecting between the plurality of upper electrodes 6 and 7, is smaller than a length of the shortest side of each of the upper electrodes 6 and 7 having a rectangular layout in planar view.

A portion of the upper surface of the lower electrode 8 on an outside of the cell array in planar view is exposed through a bottom of an opening formed on an insulation film (not shown) on the lower electrode 8. In Fig. 1, an upper surface of the lower electrode 8 in an area surrounded by the opening is illustrated as the electrode pad 4. Although only one electrode pad 4 is illustrated in Fig. 1, a plurality of electrode pads 4 may be formed. A portion of an upper surface of an electrode which is drawn out from the cell array in planar view, and is integrally formed with the upper electrodes 6 and 7 is exposed through a bottom of an opening formed on an insulation film (not shown) on the upper electrodes 6 and 7. In Fig. 1, an upper surface of the electrode in an area surrounded by the opening is illustrated as the electrode pad 3. Although only one electrode pad 3 connected with the upper electrode 7 by a metal wire is illustrated, a plurality of electrode pads 3 may be formed. The electrode pad 3 may be connected with the upper electrode 6 via the metal wire, rather than with the upper electrode 7.

Fig. 2 illustrates a cell area 1A and a condenser area 1B which are arranged in a direction parallel to the main surface of the substrate 9. That is, Fig. 2 is a cross-sectional view including the cell 2 and the condenser 5 which are adjacent to each other. Fig. 2 illustrates the cell area 1A on the left side, and illustrates the condenser area 1B on the right side. As shown in Fig. 2, the semiconductor chip 1 (see Fig. 1) includes the substrate 9, and an insulation film 13, the lower electrode 8, and insulation films 14, 15, 16, and 17 stacked on the substrate 9 in sequence. The substrate 9 includes the main surface on the upper side thereof, and a rear surface opposite to the main surface. The substrate 9 is made of silicon (Si) single crystal, for example. The substrate 9 may be a glass substrate or a sapphire substrate. The insulation films 13 to 17 are made of, for example, silicon oxide (SiO₂), silicon nitride (Si₃N₄), silicon carbide (SiC), or silicon carbonitride (SiCN).

The lower electrode 8 is formed of a conductive film such as a tungsten (W) film, a titanium nitride (TiN) film, or etc. The lower electrode 8 may have a stacking structure formed of a plurality of conductive films including a tungsten film or a titanium nitride film.

The cavity 10 is formed on the insulation film 14 in an area directly over the lower electrode 8 of the cell area 1A. The cavity 10 is a space which is covered by the insulation film 14, the insulation film 15, and the insulation film 17 that is buried in a hole opened to the insulation film 15. The cavity 10 is formed in a vacuum, for example. The cavity 10 has an upper portion and a side portion covered by the insulation film 15, and has a bottom covered by the insulation film 14. The membrane 12 which is a moving part is formed directly above the cavity 10.

That is, the membrane 12 is formed of the insulation film 15 directly over the cavity 10, the upper electrode 6, and the insulation films 16 and 17. Since the membrane 12 has spaces formed on an upper portion and a lower portion thereof, the membrane 12 moves and oscillates vertically when receiving a sound wave. The upper electrode 6 is a conductive film which has a side surface and an upper surface covered by the insulation film 16, and is formed of, for example, a tungsten (W) film, a titanium nitride (TiN) film, or etc. In addition, the upper electrode 6 may include a stacking structure which is formed of a plurality of conductive films including a tungsten film or a titanium nitride film. An interval between the lower electrode 8 and the upper electrode 6 is about 500 nm, for example. A hole vertically penetrating through a stacking film formed of the insulation films 15 and 16 is formed directly over the cavity 10, and a portion of the insulation film 17 formed on the insulation film 16 is buried in the hole. The hole and the upper electrode 6 are spaced from each other in planar view.

The material film 11 covered by the insulation film 14 and the insulation film 15 is formed on the insulation film 14 in an area directly over the lower electrode 8 of the condenser area 1B. An upper surface and a side surface of the material film 11 are covered by the insulation film 15, and a bottom surface of the material film 11 is covered by the insulation film 14. That is, the material film 11 is formed to have the same height as that of the cavity 10, and the cavity 10 and the material film 11 are formed adjacent to each other. The material film 11 is made of a material having a higher dielectric constant than that of vacuum.

The cavity 10 is a part that is formed by removing a sacrifice film made of the same material as the material film 11, and the sacrifice film remaining without being removed is the material film 11. That is, the insulation films 14-17 need to be made of a material having a selectivity with respect to an etching solution used to remove the sacrifice film, in comparison to the material film 11. The material film 11 is formed of, for example, a tetra ethyl ortho silicate (TEOS) (tetraethoxysilane) film (silicon oxide film), or a tungsten (W) film, a titanium nitride (TiN) film or a molybdenum (Mo) film. As described above, the material film 11 may be an insulation film or a conductive film. The material film 11 is formed to be spaced from both of the lower electrode 8 and the upper electrode 7. In addition, when the material film 11 is formed of a conductive film, the conductive film enters an electrically suspending state.

A film, which is a stacking film which is not moved, and is formed of the insulation film 15, the upper electrode 7, and the insulation films 16 and 17, is formed directly over the material film 11. The upper electrode 7 is formed to have the same height as that of the upper electrode 6, and the upper electrodes 6 and 7 are formed adjacent to each other. In addition, the upper electrodes 6 and 7 are integrally formed with one another, respectively. That is, the upper electrodes 6 and 7 are formed of the same conductive film, respectively. In addition, the upper electrodes 6 and 7 may be spaced from one other, respectively, but even in this case, the upper electrodes 6 and 7 are formed to have the same height and formed of the same material film, respectively. When the upper electrodes 6 and 7 are spaced from one another, respectively, the upper electrodes 6 and 7 are electrically connected with one another, respectively, by using a via and a wire, for example.

The semiconductor chip 1 (see Fig. 1) forming the ultrasonic examination device of the present embodiment includes the plurality of oscillators (cells 2) forming the capacitive ultrasonic transducer. In an operation (transmitting operation) of generating ultrasonic waves by using the capacitive ultrasonic transducer, an electrostatic force is exerted between the lower electrode 8 and the upper electrode 6 by overlappingly applying voltages of a direct current and an alternating current to the lower electrode 8 and the upper electrode 6, and the membrane 12 of each oscillator vibrates in the vertical direction in the vicinity of a resonance frequency due to equilibrium with the force of a spring of the membrane 12. Accordingly, an ultrasonic wave (ultrasonic pulse) of a few MHz is generated from the oscillator. The vertical direction and the longitudinal direction used in the present application is a perpendicular direction to the main surface of the substrate 9.

In a receiving operation by the capacitive ultrasonic transducer, the membrane 12 vibrates by a pressure of an ultrasonic wave reaching the membrane 12 of each oscillator (cell 2), and a capacitance between the lower electrode 8 and the upper electrode 6 changes, such that the ultrasonic wave can be detected. That is, the ultrasonic wave generated in the transmitting operation is focused onto an examinee (living body) and is reflected therefrom, and a displacement of an interval between the lower electrode 8 and the upper electrode 6, caused by the reflected wave, is detected as a change in the capacitance (capacitance of each oscillator). By transmitting and receiving ultrasonic waves (transmission and reception waves) using the capacitive ultrasonic transducer as described above, a tomographic image of tissue of the living body can be photographed, for example.

The semiconductor chip 1 illustrated in Fig. 1 has a capacitive type device 100 and a charging condenser 101, surrounded by the dashed line in Fig. 3, mix-mounted therein. In other words, the ultrasonic examination device of the present embodiment has a configuration in which circuits, etc. outside the area surrounded by the dashed line are connected to the semiconductor chip 1 including the capacitive type device 100. The capacitive type device 100 illustrated in Fig. 3 is the condenser (capacitive transducer, CUMT) formed of the cell 2 formed in the cell array illustrated in Fig. 1, and the charging condenser 101 illustrated in Fig. 3 is the condenser 5 illustrated in Fig. 1. As shown in Fig. 3, these condensers are connected to each other in parallel. That is, the condenser (capacitive type device 100) formed of the cell 2 includes a first terminal (first electrode) and a second terminal (second electrode), and the charging condenser 101 includes a third terminal (third electrode) and a fourth terminal (fourth electrode), and the first terminal and the third terminal are connected to each other, and the second terminal and the fourth terminal are connected to each other.

In the block diagram illustrated in Fig. 3, the first terminal at one side of the condenser which is the capacitive type device 100, and the third terminal at one side of the charging condenser 101 are connected to a direct current (DC) bias power source (direct current power source) 103 via a switch (switch element) 102. The switch 102 may control connection and disconnection (opening and closing) by using a switch controller 105. That is, the switch 102 is installed to switch on/off of the connection between the capacitive type device 100 and the charging condenser 101, and the DC bias power source 103. In other words, the switch 102 has a role of blocking the connection between the capacitive type device 100 and the charging condenser 101, and the DC bias power source 103.

The switch 102 is electrically connected to any one of the upper electrode 7 or the lower electrode 8 shown in Fig. 2. That is, the switch 102 is electrically connected to any one of the electrode pad 3 or 4 shown in Fig. 2.

The second terminal at the other side of the condenser which is the capacitive type device 100, and the fourth terminal at the other side of the charging condenser 101 are connected to a transmission power source 104 which is an AC power source (alternating current power source) for transmitting (generating a vibration). Two diodes 106 connected with each other in inverse parallel are interposed between each of the second terminal of the capacitive type device 100 and the fourth terminal of the charging condenser 101, and the transmission power source 104. The two diodes 106 are installed to prevent a signal received at the capacitive type device 100 and power supplied from the DC bias power source 103, from flowing into the transmission power source 104.

The second terminal of the capacitive type device 100 and the fourth terminal of the charging condenser 101 are connected to a DC bias blocking circuit 107. The DC bias blocking circuit 107 is connected to a transception isolation circuit 108, the transception isolation circuit 108 is connected to an amplifier circuit 109, and the amplifier circuit 109 is connected to a filter 110. The filter 110 is connected to an analogue-digital converter 111, and the analogue-digital converter 111 is connected to an image display device 112.

Hereinafter, an operation of the ultrasonic examination device will be described. When an ultrasonic wave is not transmitted or received by using the capacitive type device 100 forming the ultrasonic examination device, that is, when the capacitive type device 100 is not used, the switch 102 is closed by the switch controller 105, and a direct current voltage is applied to the charging condenser 101 from the DC bias power source 103, and accordingly, an electric charge is accumulated in the charging condenser 101. In this case, the electric charge is also accumulated in the condenser forming the capacitive type device 100. That is, by turning on the switch 102, the electric charge is accumulated in the plurality of cells 2 and the plurality of condensers 5 shown in Figs. 1 and 2. By doing so, the charging condenser 101 which is used as a battery for operating the capacitive type device 100 is charged.

In the transmitting operation of transmitting (generating a vibration) an ultrasonic wave from the capacitive type device 100, the switch 102 is opened by the switch controller 105, and enters an OFF state. A direct current voltage is applied to the capacitive type device 100 from the charging condenser 101, and an alternating current voltage is applied to the capacitive type device 100 from the transmission power source (alternate current (AC)). Accordingly, the membrane 12 (see Fig. 2) of the condenser which is the capacitive type device 100 vibrates, such that an ultrasonic wave (ultrasonic pulse) is generated from the capacitive type device 100.

In the receiving operation of receiving an ultrasonic wave in the capacitive type device 100, the switch 102 is opened by the switch controller 105 and enters an OFF state. A direct current voltage is applied to the capacitive type device 100 from the charging condenser 101. In this state, the membrane 12 vibrates by a pressure of the ultrasonic wave reaching the membrane 12 (see Fig. 2) of the condenser which is the capacitive type device 100, and a capacitance of the condenser changes, such that the ultrasonic wave can be detected.

A detected reception signal is transmitted to the amplifier circuit 109 via the DC bias blocking circuit 107 and the transception isolation circuit 108. The DC bias blocking circuit 107 includes, for example, a condenser, and has a role of preventing the direct current voltage of the DC bias power source 103 or the charging condenser 101 from being applied to the amplifier circuit 109. The transception isolation circuit 108 has a function of isolating a transmission signal and a reception signal. The amplifier circuit 109 has a role of amplifying the reception signal. The reception signal amplified by the amplifier circuit 109 is transmitted to the image display device 112 via the filter 110 and the analogue-digital converter 111. The filter 110 has a function of separating a signal of a high frequency and a signal of a low frequency from the transmitted signals, and a function of removing a noise. The analogue-digital converter 111 has a function of converting a transmitted analogue signal into a digital signal. The digitalized reception signal is displayed on the image display device 112, and is visualized.

By transmitting and receiving ultrasonic waves by using the capacitive type device 100 as described above, a tomographic image of tissue of a living body may be photographed. The present embodiment is characterized in that, in the operation of transmitting and receiving by using the capacitive type device 100, a direct current voltage is not supplied to the semiconductor chip 1 from the DC bias power source 103, and a direct current voltage is supplied to the cell 2 from the condenser 5 (see Figs. 1 and 2), which is mix-mounted in the semiconductor chip 1 along with the cell 2 (see Figs. 1 and 2) which is the capacitive type device 100. In other words, when ultrasonic waves are transmitted and received by using the capacitive type device 100, connection between the DC bias power source 103 and the semiconductor chip 1 is blocked, and a direct current voltage is applied to the capacitive type device 100 from the charging condenser 101. That is, the condenser 5 is used as a rechargeable secondary battery.

Since the condenser 5 illustrated in Fig. 2 is different from the cell 2, and is not an oscillator which transmits and receives ultrasonic waves, it is desirable that a capacitance of the condenser 5 does not change. Therefore, an insulation film or a stacking film formed of an insulation film and a conductive film is filled between the lower electrode 8 and the upper electrode 7 forming the condenser 5, and a cavity is not formed between the lower electrode 8 and the upper electrode 7. That is, the condenser formed of the cell 2 is a variable capacitance element, whereas the condenser 5 is a fixed capacitance element. The fixed capacitance element is a capacitance element which has a capacitance always fixed without being changed, even when ultrasonic waves are transmitted and received.

Accordingly, a response characteristic of the condenser forming the cell 2 when an ultrasonic wave is received, and a response characteristic of the condenser 5 when an ultrasonic wave is received are different from each other. That is, the response characteristic of the condenser 5 is lower than the response characteristic of the cell 2. In other words, when a predetermined ultrasonic wave is received by the semiconductor chip 1 (see Fig. 1), a sensitivity (receive sensitivity) of one condenser 5 is lower than a sensitivity (receive sensitivity) of one cell 2.

Accordingly, an extra reception signal (noise) is prevented from being generated in the condenser 5 in the receiving operation. That is, an unnecessary response can be prevented from being generated by installation of the condenser 5.

It is desirable to enhance charging performance in the condenser 5 used as a secondary battery. Therefore, it is desirable that the material film 11 illustrated in Fig. 2 is a conductive film from the perspective of increasing the capacity of the condenser 5. In this case, a sum distance of a distance between the upper electrode 7 of the condenser 5 and the conductive film, and a distance between the lower electrode 8 of the condenser 5 and the conductive film may be made small, in comparison to a distance between the upper electrode 6 and the lower electrode 8 of the cell 2. Therefore, the capacitance of the condenser 5 can be increased. The capacitance of the condenser 5 can be further increased by forming the material film 11 by a material having a higher dielectric constant than that of vacuum. In the present embodiment, the capacitance of one condenser 5 is greater than the capacitance of the condenser forming one cell 2. Therefore, when the switch 102 shown in Fig. 3 is closed and the cell 2 and the condenser 5 are charged by the DC bias power source 103, respectively, an electric charge accumulated in one condenser 5 is greater than an electric charge accumulated in one cell 2.

The condenser 5 may be formed in the same configuration as that of the cell 2 such that the material film 11 may be removed and a cavity may be formed between the upper electrode 7 and the lower electrode 8. However, in this case, there is a need to prevent the stacking film, including the upper electrode 7 on the cavity, from operating like the membrane 12, in order to prevent an unnecessary response from being generated in the condenser 5 during the receiving operation. To achieve this, for example, a whole width of the condenser 5 may be made smaller than a whole width of the cell 2, and an area of the condenser 5 in planar view may be made small. Accordingly, a vibration of the stacking film including the upper electrode 7 can be prevented, and thus a noise can be prevented from being generated in the condenser 5. Even when the condenser 5 includes a cavity between upper and lower electrodes as described above, responding performance of the condenser 5 having a smaller width in the transverse direction than that of the cell 2 is lower than the response characteristic of the cell 2. The transverse direction in the present application is a horizontal direction parallel to the upper surface of the substrate 9.

### <Manufacturing Method of the Ultrasonic Examination Device of the Present Embodiment>

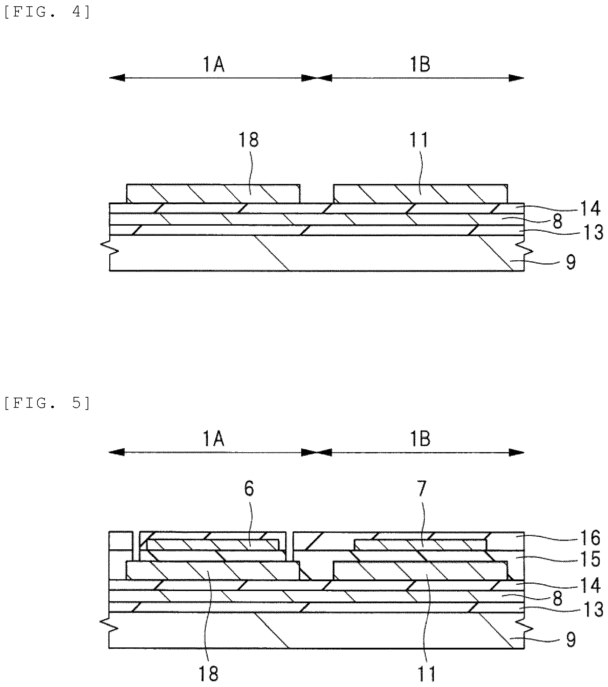

Hereinafter, a method for manufacturing the semiconductor chip forming the ultrasonic examination device of the present embodiment will be described with reference to Figs. 4 to 7. Figs. 4 to 7 are cross-sectional views of the semiconductor chip forming the ultrasonic examination device in the process of manufacturing according to the present embodiment.

As shown in Fig. 4, the substrate 9 is prepared. The substrate 9 is a semiconductor substrate (semiconductor wafer) formed of, for example, single crystal silicon (Si). The insulation film 13, the lower electrode 8, the insulation film 14, and the material film 11 are formed (stacked) on the substrate 9 in sequence. The insulation films 13, 14 are made of, for example, silicon oxide (SiO₂), silicon nitride (Si₃N₄), silicon carbide (SiC), or silicon carbonitride (SiCN). The lower electrode 8 is formed of a conductive film such as a tungsten (W) film, a titanium nitride (TiN) film, or etc. The lower electrode 8 may include a stacking structure formed of a plurality of conductive films including a tungsten film or a titanium nitride film. The material film 11 may be formed of, for example, a TEOS film (silicon oxide film), a tungsten (W) film, a titanium nitride (TiN), or a molybdenum (Mo) film.

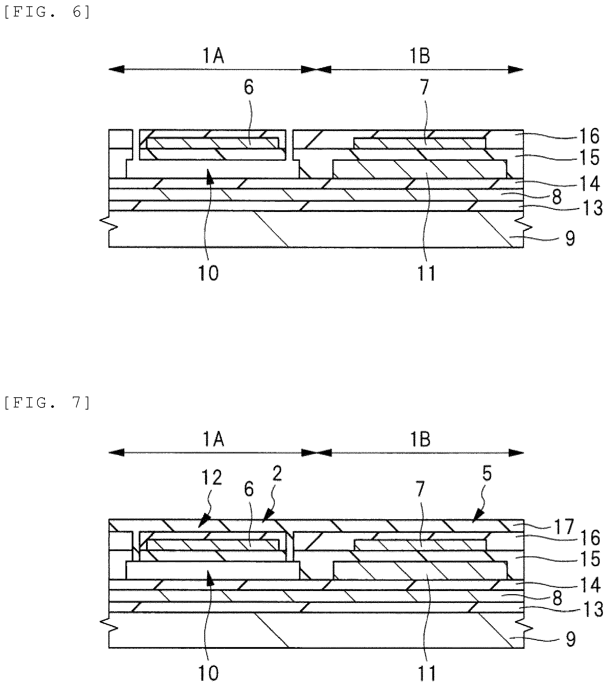

The insulation film 14 is made of a material having a selectivity with respect to an etching solution used to remove the material film 11 in an etching process, which will be described below with reference to Fig. 6. The insulation films 13, 14 may be formed by chemical vapor deposition (CVD), for example. The lower electrode 8 may be formed by sputtering, for example. When the material film 11 is formed of a TEOS film, the material film 11 may be formed by CVD, for example, and, when the material film 11 is formed of a metal film, the material film 11 may be formed by sputtering, for example.

Subsequently, the material film 11 is processed (sputtering) by photolithography technology and etching, and accordingly, a portion of the upper surface of the insulation film 14 is exposed. Herein, in the area including the cell area 1A and the condenser area 1B, the material film 11 remains as a plurality of patterns arranged in the form of a matrix. The plurality of patterns are spaced from one another , and a planar shape of each of the plurality of patterns is a rectangle. Accordingly, in the cell area 1A, a plurality of sacrifice films 18 formed of the material film 11 are arranged in the form of matrix in planar view. In the condenser area 1B, the plurality of patterns of the material film 11 remain adjacent to the plurality of sacrifice films 18 arranged on an edge of the cell area 1A. The sacrifice films 18 are sacrifice patterns which are removed in the next process which will be described with reference to Fig. 6, and do not remain in the ultrasonic examination device after completion.

Next, as shown in Fig. 5, the insulation film 15 is formed on the insulation film 14 to cover the sacrifice films 18 and the material films 11, and then, an upper surface of the insulation film 15 is smoothed by polishing by chemical mechanical polishing (CMP), for example. The insulation film 15 is made of, for example, silicon oxide (SiO₂), silicon nitride (Si₃N₄), silicon carbide (SiC), silicon carbonitride (SiCN), or etc. The insulation film 15 may be formed by CVD, for example.

Subsequently, a conductive film is formed on the insulation film 15 by sputtering, for example. The conductive film may be formed of, for example, a tungsten (W) film, a titanium nitride (TiN) film, or etc. In addition, the conductive film may have a stacking structure formed of a plurality of conductive films including a tungsten film or a titanium nitride (TiN) film. Subsequently, the conductive film is processed (patterning) by photolithography technology and etching, and accordingly, a portion of the upper surface of the insulation film 15 is exposed.

Herein, the upper electrode 6 formed of the conductive film are arranged in the cell area 1A in the form of a matrix, and the plurality of upper electrodes 7 formed of the conductive film are formed in the condenser area 1B alongside of the upper electrodes 6. In addition, a metal wire which is a wire formed of the conductive film is formed to connect the upper electrodes 6 which are spaced from each other and are adjacent to each other, the upper electrode 6 and the upper electrode 7 which are spaced from each other and are adjacent to each other, and the upper electrodes 7 which are spaced from each other and are adjacent to each other, respectively. That is, by patterning one the conductive film, the upper electrodes 6, the upper electrodes 7, and the metal wire, which are integrally formed with one another, are formed. Herein, an extraction electrode (not shown) formed of the conductive film is formed on an outside of the area in which the upper electrodes 6 and 7 are arranged in the form of an array. The extraction electrode is a portion where the electrode pad 3 (see Fig. 1) is formed afterward.

The planar shape of each of the upper electrodes 6 and 7 is a rectangle. In addition, one upper electrode 6 is formed directly over each of the plurality of sacrifice films 18 in the cell area 1A, and one upper electrode 7 is formed directly over each of the plurality of material films 11 in the condenser area 1B.

Subsequently, the insulation film 16 is formed on the insulation film 15 to cover the upper electrodes 6 and 7, the metal wire, and the extraction electrode, and then, an upper surface of the insulation film 16 is smoothed by polishing by CMP, for example. The insulation film 16 is made of, for example, silicon oxide (SiO₂), silicon nitride (Si₃N₄), silicon carbide (SiC), silicon carbonitride (SiCN), or etc. The insulation film 16 may be formed by CVD, for example.

Subsequently, a hole penetrating through the stacking film formed of the insulation films 15 and 16, formed directly over each of the plurality of sacrifice films 18 in the cell area 1A, is formed by photolithography technology and dry etching. Herein, the hole is formed adjacent to the upper electrode 6. The upper surface of the sacrifice film 18 is exposed to a bottom surface of the hole. One of the features of the present embodiment is that the hole is not formed directly over the material film 11. Accordingly, after the etching process is performed, the sacrifice film 18 has a portion thereof exposed, but the material film 11 is not exposed.

Next, as shown in Fig. 6, the sacrifice film 18 is removed by wet etching, for example. Accordingly, the cavity 10 is formed in the area from which the sacrifice film 18 is removed. Herein, wet-etching is performed under the condition that there is a selectivity with respect to the insulation films 14-16, and the sacrifice film 18 is selectively removed. In this case, since the whole material film 11 is covered by the insulation films 14-16, the material film 11 is not removed by etching and remains.

Next, as shown in Fig. 7, the insulation film 17 is formed on the insulation film 16 including the inside of the hole. The insulation film 17 is made of, for example, silicon oxide (SiO₂), silicon nitride (Si₃N₄), silicon carbide (SiC), silicon carbonitride (SiCN), or etc. The insulation film 17 may be formed by CVD, for example. Accordingly, the inside of the hole directly over the cavity 10 is buried by a portion of the insulation film 17, but the cavity 10 is not buried and remains. For example, the cavity 10 is in a vacuum state, for example, and is sealed.

Subsequently, an opening (not shown) is formed to penetrate through the insulation films 16, 17 positioned outside the cell area 1A and the condenser area 1B in planar view, and to expose the upper surface of the extraction electrode, such that the electrode pad 3 (see Fig. 1) is formed on the upper surface of the extraction electrode on the bottom of the opening. An opening (not shown) is formed to penetrate through the insulation films 14-17 positioned outside the cell area 1A and the condenser area 1B in planar view, and to expose the upper surface of the lower electrode 8, such that the electrode pad 4 (see Fig. 1) is formed on the bottom of the opening.

Through the above-described process, the lower electrode 8, the cavity 10 on the lower electrode 8, and the membrane 12 including the insulation films 15-17 and the upper electrode 6 on the cavity 10 are formed in the cell area 1A. The lower electrode 8 and the upper electrode 6 stacked in the cell area 1A in the longitudinal direction, and the cavity 10 between the lower electrode 8 and the upper electrode 6 form the cell 2 which is the condenser. In addition, the condenser 5 formed of the lower electrode 8 and the upper electrode 7 on the lower electrode 8 is formed in the condenser area 1B. A cavity is not formed between the lower electrode 8 and the upper electrode 7 forming the condenser 5, and the stacking film formed of the insulation films 14 and 15 and the material film 11 is formed from the upper surface of the lower electrode 8 to the lower surface of the upper electrode 7.

Subsequently, the semiconductor wafer including the substrate 9 is divided by cutting by a dicing process, and the plurality of semiconductor chips 1 (see Fig. 1) are obtained. Accordingly, the semiconductor chip 1 forming the ultrasonic examination device of the present embodiment is formed. Thereafter, the ultrasonic examination device of the present embodiment is completed by connecting a bonding wire to the electrode pads 3 and 4 on the main surface of the semiconductor chip 1, and electrically connecting the semiconductor chip 1 the power sources and the circuits shown in Fig. 3 via the bonding wire.

### <Effects of the Present Embodiment>

Hereinafter, effects of the ultrasonic examination device and the manufacturing method thereof of the present embodiment will be described.

When ultrasonic waves are transmitted and received by using the ultrasonic examination device, and an image is obtained, there is a need to always apply a direct current voltage to the ultrasonic transducer. In an ultrasonic examination device which uses an ultrasonic transducer in a blood vessel catheter, the direct current voltage of 200 V is used, but it is apprehended that a current leaks into a blood vessel when a short circuit breakdown occurs, and there is a need to take secure measures against a direct current bias voltage. The above-described problem of the current leakage is not limited to the case where the ultrasonic examination device is used in the blood vessel catheter, and is easy to arise in the case where the probe of the ultrasonic examination device is used in a wearable sensor worn on a living body, and the case where the probe of the ultrasonic examination device is used as an ultrasonic probe of an ultrasonic echo diagnostic device that is brought into contact with epidermis from the outside of a living body, and examines the internal structure of the living body.

Herein, it may be considered that, in an ultrasonic probe having a first semiconductor chip mounted therein and including an ultrasonic transducer, another second semiconductor chip having a charging condenser mounted therein is mounted, and in a transmitting and receiving operation of the ultrasonic transducer, a direct current voltage is not applied to the first semiconductor chip from a DC bias power source, and a direct current voltage is applied to the first semiconductor chip from the charging condenser. In this case, when a short circuit breakdown occurs in the ultrasonic probe, it may be possible to prevent a current from continuously leaking into a living body.

However, when the second semiconductor chip having the charging condenser formed therein is mounted in the ultrasonic probe, separately from the first semiconductor chip, a parasitic capacitance in a voltage applying path from the charging condenser in the second semiconductor chip to a cell array of the first semiconductor chip becomes greater. To this end, an electric charge is easily discharged from the charging condenser, and it is difficult to perform the transmitting and receiving operation using the ultrasonic transducer for a long time.

When the second semiconductor chip having the charging condenser formed therein is mounted in the ultrasonic probe, there is a problem that the ultrasonic probe is large. In particular, when a probe for examination is required to be miniaturized like a blood vessel catheter, this problem is noticeable.

In contrast, in the present embodiment, the cell (oscillator) 2 of the ultrasonic transducer (CMUT), and the condenser 5 for charging the cell 2 are formed in one semiconductor chip 1 as shown in Figs. 1 and 2. As described with reference to Fig. 3, when ultrasonic waves are transmitted and received by using the capacitive type device 100, a direct current voltage can be applied to the capacitive type device 100 from the charging condenser 101, which is charged in advance by using the DC bias power source 103, and the transmitting and receiving operation can be performed, although a direct current voltage is not applied to the semiconductor chip 1 from the DC bias power source 103. That is, when a short circuit breakdown occurs in the ultrasonic probe, it is possible to prevent a current from continuously leaking into a living body from the semiconductor chip 1.

Since the cell 2 and the condenser 5 are mix-mounted in one semiconductor chip 1 as shown in Figs. 1 and 2, a voltage applying path from the condenser 5 to the cell 2 can be shortened. That is, a parasitic capacitance generated in the voltage applying path from the charging condenser to the capacitive type device can be greatly reduced, in comparison to the case where the first semiconductor chip including the ultrasonic transducer, and the second semiconductor chip including the charging condenser are mounted in the ultrasonic probe as described above. Accordingly, electric discharge from the charging condenser can be suppressed, and a continuous operation time of the ultrasonic probe can be extended. Accordingly, performance of the ultrasonic examination device can be enhanced.

Since the cell 2 and the condenser 5 are mix-mounted in one semiconductor chip 1, the ultrasonic probe can be miniaturized. Accordingly, performance of the ultrasonic examination device can be enhanced.

In addition, in the present embodiment, the cell 2 and the condenser 5 illustrated in Figs. 1 and 2 have almost the same structures and are arranged, and a difference in the structures therebetween is only the presence/absence of the material film 11. That is, the cell 2 has the cavity 10 formed therein by removing the sacrifice film 18 (see Fig. 5) between the upper and lower electrodes, whereas, in the condenser area 1B, the material film 11 is not removed and remains. As described above, the cell 2 and the condenser 5 can be separately manufactured simply by leaving the material film 11 in a certain area (condenser area 1B) without removing the same. Accordingly, the condenser 5 can be additionally formed in the semiconductor chip 1 without increasing the number of processes, in comparison to the case where the cell 2 is formed in the semiconductor chip 1 and the condenser 5 is not formed. That is, a manufacturing cost of the ultrasonic examination device including the semiconductor chip 1 can be reduced.

### <Variation>

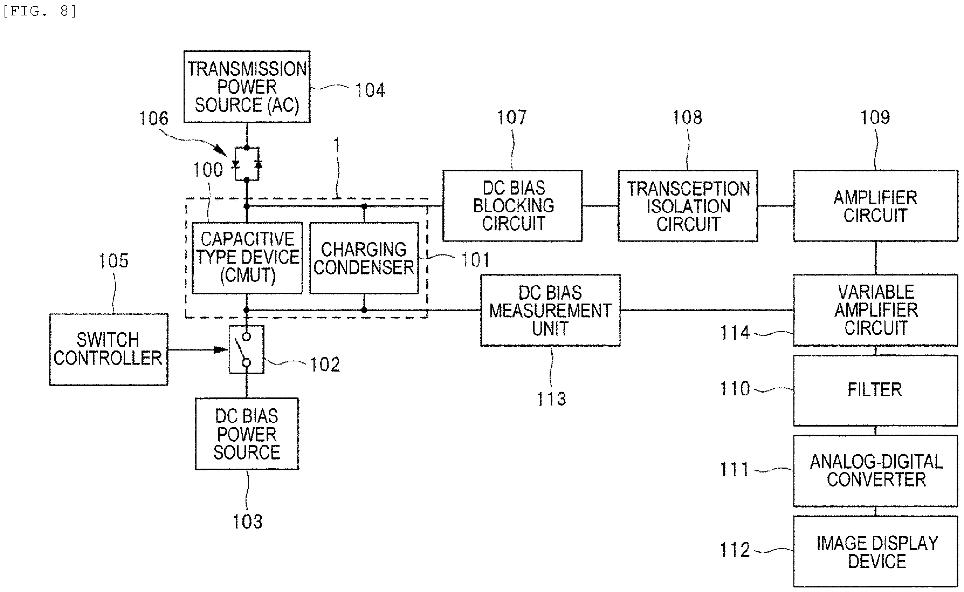

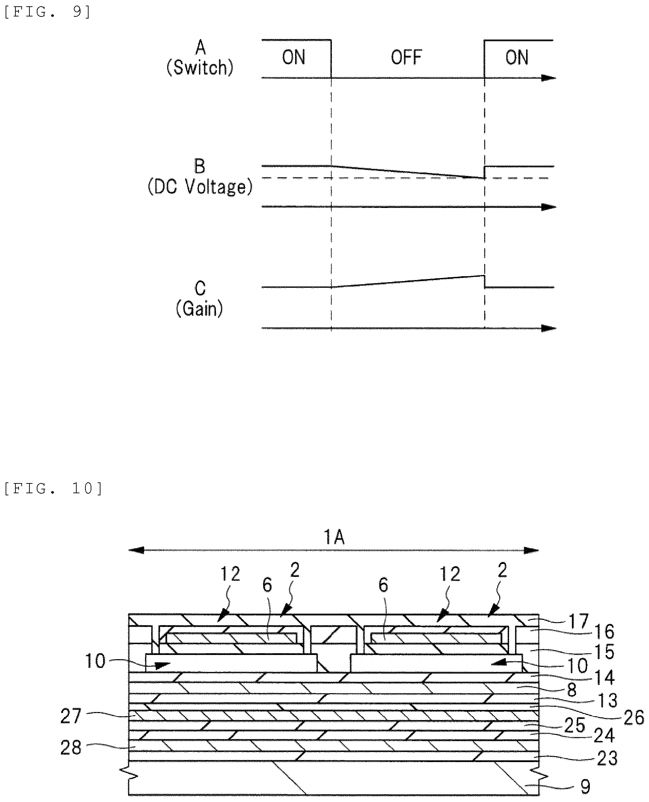

Fig. 8 illustrates a variation of the ultrasonic examination device of the present embodiment, the variation forming part of the claimed scope of the present invention. FIG. 8 is a block diagram illustrating a configuration of the ultrasonic examination device according to a variation of the present embodiment. Fig. 9 illustrates a timing chart illustrating an operation of the ultrasonic examination device of the present variation. In Fig. 9, the chart A illustrates ON-OFF states of the switch 102 (see Fig. 8), the chart B illustrates a DC bias voltage applied to the capacitive type device, and the chart C illustrates a gain of a reception signal by a variable amplifier circuit.

The present variation differs from the configuration of Fig. 3 in that a mechanism for measuring a DC bias voltage of the capacitive type device 100 is provided, and a mechanism for adjusting a signal gain according to the measured DC bias voltage is provided.

That is, as shown in Fig. 8, the first terminal of the capacitive type device 100 and the third terminal of the charging condenser 101, connected to the DC bias power source 103 via the switch 102, are connected to a DC bias measurement unit 113, and the DC bias measurement unit 113 is connected to a variable amplifier circuit 114. The variable amplifier circuit 114 is inserted between the amplifier circuit 109 and the filter 110. That is, the amplifier circuit 109 is connected to the transception isolation circuit 108 and the variable amplifier circuit 114, and the filter 110 is connected to the variable amplifier circuit 114 and the analog-digital converter 111. The amplifier circuit 109 and the filter 110 are connected to each other via the variable amplifier circuit 114.

The DC bias measurement unit 113 is a unit for measuring a direct current voltage (DC bias voltage) applied to the capacitive type device 100, and in particular, is a unit for measuring the direct current voltage when the switch 102 enters an OFF state. The chart B of Fig. 9 illustrates a change in the direct current voltage over time. In the ultrasonic examination device of the present variation forming part of the claimed scope, the electric charge accumulated in the charging condenser 101 is reduced by operating the capacitive type device 100 after the switch 102 shown in Fig. 8 is turned off (see charts A, B of Fig. 9). As a result, as shown in the chart B of Fig. 9, the DC bias voltage applied to the capacitive type device 100 is gradually reduced. The DC bias measurement unit 113 shown in Fig. 8 detects the reduction of the DC bias voltage, and transmits the result thereof to the variable amplifier circuit 114.

The variable amplifier circuit 114 amplifies a reception signal received from the amplifier circuit 109 according to the result measured at the DC bias measurement unit 113, and accordingly, performs calibration (adjustment) to make a receive sensitivity constant. The variable amplifier circuit 114 gradually increases the gain of the reception signal (magnification ratio) according the DC bias voltage gradually reduced (see the chart C of Fig. 9). However, when the DC bias voltage is lower than a predetermined threshold value as shown in the chart B of Fig. 9, the switch 102 shown in Fig. 8 is turned on, and the DC bias voltage is applied to the capacitive type device 100 from the DC bias power source 103.

In the present invention, since the capacitive type device 100 is disconnected from the DC device power source 103 during the operation of the capacitive type device 100, the DC bias voltage applied to the capacitive type device 100 cannot be always maintained at a constant level. However, by controlling the gain of the reception signal by using the DC bias measurement unit 113 and the variable amplifier circuit 114, the receive sensitivity can be prevented from being changed.

Although it is illustrated that the variable amplifier circuit 114 is inserted between the amplifier circuit 109 and the filter 110, the variable amplifier circuit 114 may be inserted into other positions as long as the variable amplifier circuit 114 is between the second terminal of the capacitive type device 100 and the analogue-digital converter 111.

### (Second Embodiment)

Although it is illustrated in the first embodiment that the charging condenser is mix-mounted in the same semiconductor chip alongside of the cell of the CMUT, the charging condenser mix-mounted in the semiconductor chip along with the cell of the CMUT may be installed under the cell in the semiconductor chip. In the present embodiment, a cell of a CMUT and a charging condenser which are stacked one on another in the same semiconductor chip will be described with reference to Fig. 10. Fig. 10 is a cross-sectional view illustrating a semiconductor chip forming an ultrasonic examination device of the present embodiment. In Fig. 10, two cells forming a cell array of a CMUT are arranged unlike in Fig. 2. That is, only the cell area 1A is illustrated in Fig. 10.

As shown in Fig. 10, the structure of the cell 2 is the same as in the first embodiment. That is, an insulation film 13, a lower electrode 8, an insulation film 14, an insulation film 15, an insulation film 16, and an insulation film 17, which are stacked on an upper portion of a substrate 9 in sequence, are formed, and the cell 2 is formed of the lower electrode 8, a cavity 10 formed between the insulation film 14 and the insulation film 15, and an upper electrode 6 formed between the insulation film 15 and the insulation film 16.

However, a condenser 35 is formed directly under the cell 2 between the substrate 9 and the insulation film 13. That is, an insulation film 23, a lower electrode 28, an insulation film 24, an insulation film 25, an upper electrode 27, and an insulation film 26 are stacked on the substrate 9 in sequence. The lower electrode 28 and the upper electrode 27 form the condenser 35, that is, the charging condenser 101 shown in Fig. 3. An upper surface of the insulation film 26 is smoothed, and the insulation film 13 is formed on the insulation film 26.

The lower electrode 28 of the condenser 35 and the lower electrode 8 of the cell 2 are electrically connected with each other, and the upper electrode 27 of the condenser 35 and the upper electrode 6 of the cell 2 are electrically connected with each other. That is, the condenser forming the cell 2, and the condenser 35 are connected to each other in parallel. Each of the upper electrode 27 and the lower electrode 28 is widely formed to cover almost the whole semiconductor chip, such that each of the upper electrode 27 and the lower electrode 28 overlaps the cell array (cell area 1A) having the plurality of cells 2 arranged therein in planar view. That is, a capacity of the condenser 35 is greatly larger than a capacity of each cell 2.

The upper electrode 27 and the lower electrode 28 are designed to have a short distance therebetween from the perspective of increasing the capacity of the condenser 35. That is, the distance between the upper electrode 27 and the lower electrode 28 is smaller than a distance between the upper electrode 6 and the lower electrode 8. In other words, a film thickness of the insulation film (herein, a stacking film of the insulation films 24, 25) between the upper electrode 27 and the lower electrode 28 is smaller than the distance between the upper electrode 6 and the lower electrode 8. Accordingly, since the distance between the upper and lower electrodes of the condenser 35 can be reduced, in comparison to the condenser 5 (see Fig. 2) of the first embodiment, the capacity of the condenser 35 can be increased. As a result, an operation time of the capacitive type device disconnected from the DC bias power source can be extended.

In the present embodiment, a material and a film forming method of each of the lower electrode 28 and the upper electrode 27 are the same as the material and the film forming method of the lower electrode 8. In addition, the insulation films 23-26 may be formed in the same method as the insulation films 13-16.

In the present embodiment, it is not necessary to form the charging condenser 35 in the same manufacturing process as the cell 2. Accordingly, the distance between the upper and lower electrodes of the condenser 35 can be reduced. In addition, the condenser 35 can be formed in a wide area overlapping the cell array in planar view, rather than on the periphery of the cell area. Accordingly, it is easy to increase the capacity of the condenser 35 in comparison to the first embodiment.

Since it is not necessary to install the charging condenser on the periphery of the cell array, the area of the semiconductor chip can be reduced in comparison to the first embodiment.

As a variation of the present embodiment, a cavity (gap) may be formed between the insulation film 24 and the insulation film 25 in the same way as the cavity 10. In this case, a plurality of cavities may be arranged in the same way as the cavity 10, or the cavity may be formed to be large so as to overlap the whole cell array in planar view. When one cavity having an enough size to overlap the whole cell array is formed, a plurality of supports for supporting between the insulation film 24 and the insulation film 25 may be arranged in the cavity to prevent the insulation film on the upper portion of the cavity from moving and to prevent the cavity from being worn out.

In a combination of the first embodiment and the present embodiment, the condenser 35 directly under the cell 2, and the condenser 5 (see Figs. 1 and 2) adjacent to the cell 2 may be formed, and the condensers 5, 35 are connected with each other in parallel. In this case, there is a possibility that the effect of reducing the area of the semiconductor chip 1 is not obtained, in comparison to the first embodiment, but the capacity of the charging condenser can be further increased.

### (Third Embodiment)

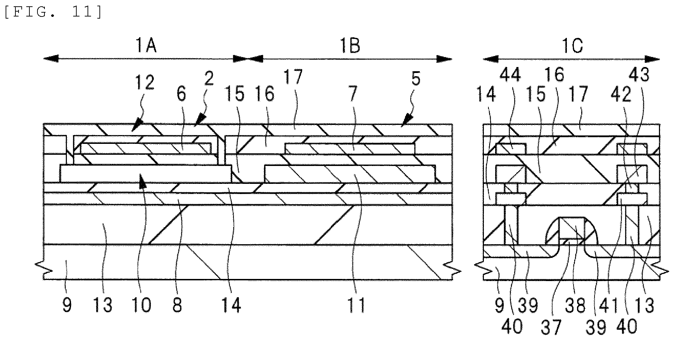

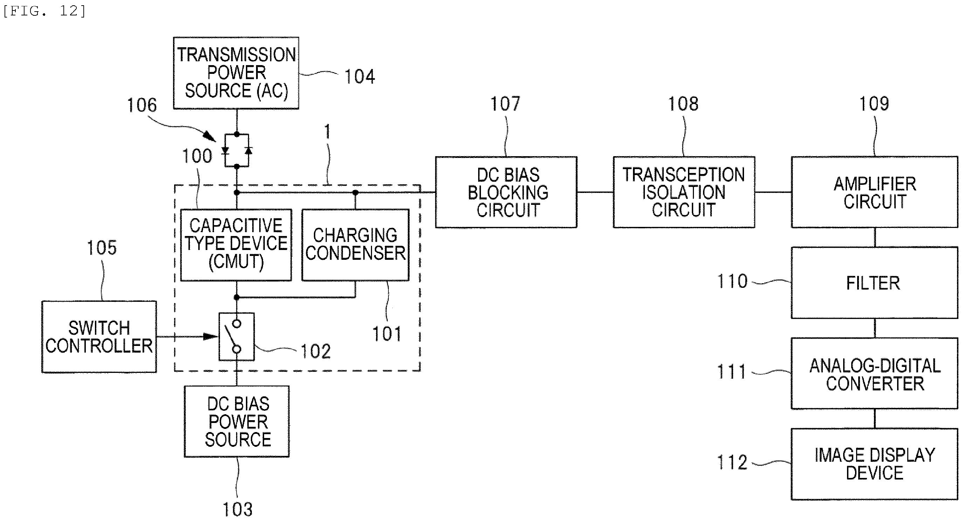

In the present embodiment, the switch 102 shown in Fig. 3 being mounted in the semiconductor chip 1 will be described with reference to Figs. 11 and 12. Herein, a case where the switch 102 is formed as a semiconductor element on a semiconductor substrate will be described. Fig. 11 is a cross-sectional view illustrating a semiconductor chip forming an ultrasonic examination device of the present embodiment. Fig. 12 is a block diagram illustrating a configuration of the ultrasonic examination device of the present embodiment.

As shown in Fig. 12, a semiconductor chip 1 of the present embodiment has a capacitive type device 100 and a charging condenser 101 mounted therein in the same way as in the first embodiment, and furthermore, has a switch 102 mounted therein and connected to the capacitive type device 100 and the charging condenser 101. As shown in Fig. 11, the switch 102 is configured by a transistor which is a semiconductor element. In Fig. 11, a cell area 1A and a condenser area 1B of the semiconductor chip are illustrated on the left side, and a switch area 1C of the semiconductor chip is illustrated on the right side.

The structures of the cell area 1A and the condenser area 1B illustrated in Fig. 11 are the same as in the first embodiment. In contrast, in the switch area 1C shown in Fig. 11, a transistor which is a metal insulator semiconductor field effect transistor (MISFET) is formed. That is, a gate electrode 38 is formed on the substrate 9 of the switch area 1C via a gate insulation film 37, and a pair of source-drain areas 39 are formed on an upper surface of the substrate 9 which is the semiconductor substrate at sides of the gate electrode 38. The source-drain areas 39 and the gate electrode 38 form the transistor. The gate insulation film 37 is formed of, for example, a silicon oxide film, and the gate electrode 38 is formed of, for example, a polysilicon film. The source-drain areas 39 are semiconductor areas which are formed by introducing an n-type impurity or a p-type impurity into the upper surface of the substrate 9.

An insulation film 13 is formed to cover the source-drain areas 39 and the gate electrode 38, plugs 40 which are conductive connection portions penetrating through the insulation film 13 are electrically connected to upper surfaces of the source-drain areas 39 and the gate electrode 38, respectively. The plug 40 on the gate electrode 38 is formed in an area which is not illustrated. A silicide layer (not shown) is formed between the plug 40 and the source-drain area 39 and between the plug 40 and the gate electrode 38. The plug 40 is formed of, for example, a tungsten (W) film.

Upper surfaces of the plugs 40 are smoothed to be within the same surface as the upper surface of the insulation film 13, and the upper surfaces of the plugs 40 are connected to lower surfaces of wires 41 formed on the insulation film 13 and covered by an insulation film 14. The wires 41 are made of the same material as that of the lower electrode 8, for example. Vias 42 which are conductive connection portions penetrating through the insulation film 14 are connected to the upper surfaces of the wires 41. The via 42 is formed of, for example, a tungsten (W) film. Upper surfaces of the vias 42 are smoothed to be within the same surface as the upper surface of the insulation film 14, and the upper surfaces of the vias 42 are connected to lower surfaces of wires 43 which are formed on the insulation film 14 and are covered by an insulation film 15. The wire 43 is formed of, for example, the same conductive film as the material film 11. In the switch area 1C, wires 44 which are covered by an insulation film 16 are formed on the insulation film 15. The wires 44 and the wires 43 are connected with each other by vias (not shown) penetrating through the insulation film 15. The wires 44 are made of the same material as the upper electrodes 6 and 7.

The transducer is a switch element which can switch between an ON state and an OFF state by the switch controller 105 shown in Fig. 12, and one of the source-drain areas 39 is electrically connected to the first electrode (for example, the lower electrode 8) of the cell 2 and the third electrode (for example, the lower electrode 8) of the condenser 5, and the other one of the source-drain areas 39 is electrically connected to the DC bias power source 103. The gate electrode 38 is electrically connected to the switch controller 105.

In the present embodiment, the switch 102 is mounted in the semiconductor chip 1 along with the capacitive type device 100 and the charging condenser 101, such that a voltage applying path between the switch 102 and these condensers can be shortened. Accordingly, a parasitic capacitance between the switch 102 and these condensers can be reduced, and a leak path can be reduced. That is, since electric discharge from the charging condenser 101 can be prevented, it is possible to operate the capacitive type device 100 for a longer time.

### <Variation 1>

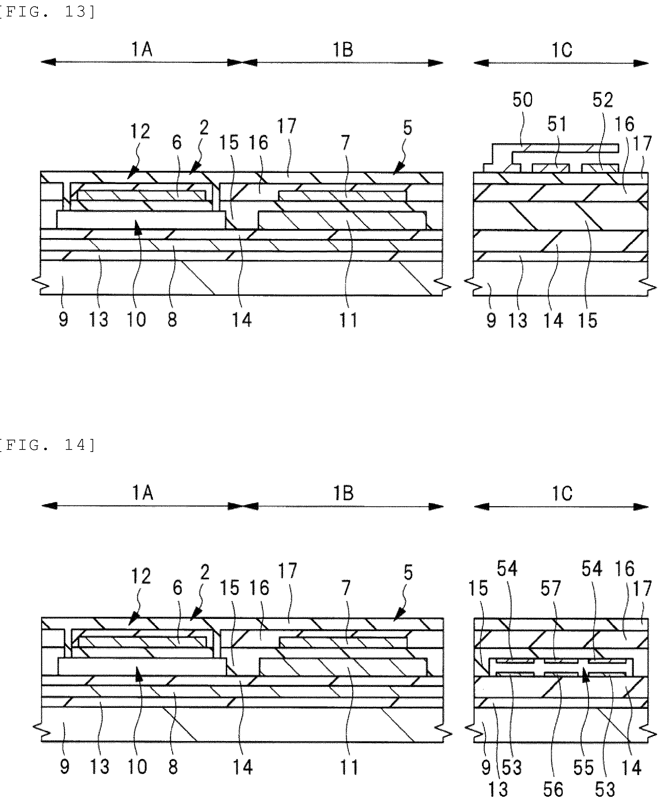

In the present variation, a switch which is mounted in a semiconductor chip and is configured by a mechanical switch formed by MEMS technology, rather than by a semiconductor element, will be described with reference to Fig. 13. Fig. 13 is a cross-sectional view illustrating a semiconductor chip forming an ultrasonic examination device of the present embodiment.

As shown in Fig. 13, a structure of a switch area 1C of the present variation is different from the structure of the switch area 1C illustrated in Fig. 11. Specifically, in the switch area 1C of the present variation, a switch which is a semiconductor element is not formed, and insulation films 13-17 are stacked on a substrate 9 in sequence. A contact switching switch (MEMS switch element) is formed on the insulation film 17.

The contact switching switch are formed of three terminals, and these terminals may be regarded as a gate, a source, and a drain. In the switch area 1C of Fig. 13, the contact switching switch in an OFF state is illustrated. For example, a metal plate (electrode) 50 functioning as a source has one end fixed to an upper surface of the insulation film 17, and the other end upwardly spaced from the upper surface of the insulation film 17 and floating. That is, a lower surface of the metal plate 50 faces the upper surface of the insulation film 17, and one end of the metal plate 50 in the transverse direction is fixed to the upper surface of the insulation film 17, whereas the other portions of the metal plate 50 are not fixed to any position. An electrode 51 functioning as a gate and an electrode 52 functioning as a drain are arranged on an upper surface of the insulation film 17 alongside each other directly under the metal plate 50. The electrodes 51 and 52 are spaced from each other, and an upper surface of each of the electrodes 51 and 52, and the lower surface of the metal plate 50 are spaced from each other. The electrode 51 is arranged on a position close to the fixing portion between the metal plate 50 and the insulation film 17, in comparison to the electrode 52.

Herein, when a direct current voltage is applied to the electrode 51, an electrostatic force pulling down the metal plate 50 is generated. Accordingly, the end of the metal plate 50 that is not fixed moves downward, and comes into contact with the electrode 52, such that the metal plate 50 and the electrode 52 are electrically connected to each other, and the contact switching switch enters an ON state. When the voltage applied to the electrode 51 which is a gate is 0V, the attractive force between the electrode 51 and the metal plate 50 disappears, and thus the metal plate 50 is separated from the electrode 52 and the contact switching switch returns to the OFF state.

In the present variation, the contact switching switch is a switch which is used as the switch 102 mounted in the semiconductor chip 1 shown in Fig. 12. As described above, the switch 102 connected between the capacitive type device 100 and the charging condenser 101, and the DC bias power source 103, is formed in the semiconductor chip 1. Accordingly, as described above with reference to Figs. 11 and 12, the voltage applying path between the switch 102 and the capacitive type device 100 and the charging condenser 101 can be shortened. Accordingly, a parasitic capacitance between the switch 102 and the capacitive type device 100 and the charging condenser 101 can be reduced, and a leak path can be reduced.

Since the contact switching switch mechanically spaces a source terminal and a drain terminal from each other in an OFF state, a leak current is rarely generated. Accordingly, since the occurrence of a leak current can be suppressed in the OFF state, in comparison to the case where the switch 102 is formed by a semiconductor element (see Fig. 11), power saving of the semiconductor chip can be achieved. That is, it is possible to extend an operation time of the capacitive type device 100 using the charging condenser 101 as a power source.

### <Variation 2>

In the first variation, a switch of a cantilever type having one end of a terminal (metal plate) fixed has been described. In the present variation, a switch which is mounted in the semiconductor chip, and is a mechanical switch (MEMS switch element) formed by using MEMS technology, and has both ends of a movable terminal fixed will be described with reference to Fig. 14. Fig. 14 is a cross-sectional view illustrating a semiconductor chip forming an ultrasonic examination device of the present embodiment. In Fig. 14, an MEMS switch in an OFF state is illustrated.

As shown in Fig. 14, a structure of a switch area 1C of the present variation is different from the structure of the switch area 1C shown in Figs. 11 and 13. Specifically, a switch which is a semiconductor element is not formed in the switch area 1C of the present variation, and insulation films 13-17 are stacked on a substrate 9. Herein, a cavity 55 is formed on the insulation film 14 in the same way as the cavity 10 of the cell area 1A. An electrode 56 and a pair of electrodes 53 having the electrode 56 disposed therebetween are formed on a lower surface of the inside of the cavity 55, that is, in contact with an upper surface of the insulation film 14. In addition, an electrode 57 and a pair of electrodes 54 having the electrode 57 disposed therebetween are formed on an upper surface of the inside of the cavity 55, that is, in contact with a lower surface of the insulation film 15.

The electrode 56 and the electrode 57 are arranged to overlap each other in planar view, and are spaced from each other. The electrodes 53 and the electrodes 54 are arranged to overlap each other in planar view, and are spaced from each other. An stacking film formed of the insulation films 15 to 17, directly over the cavity 55, is a membrane (flexible film) . The MEMS switch of the present variation applies a direct current voltage to one of the electrodes 53 or 54, and applies, for example, 0V to the other one, thereby generating an electrostatic force pulling each other between the electrodes 53 and the electrodes 54. Accordingly, a center of the membrane is bent downward and the electrodes 56, 57 come into contact with each other and are electrically connected with each other, such that the MEMS switch enters an ON state. When the voltage applied to the electrodes 53 or 54 is 0V, the electrodes 56, 57 are spaced from each other again, and the MEMS switch enters an OFF state.

In the present variation, the same effect as in the first variation of the present embodiment can be obtained by mounting the mechanical MEMS switch element including the electrodes 56 and 57 spaced from each other during the OFF state in the semiconductor chip.

### (Fourth Embodiment)

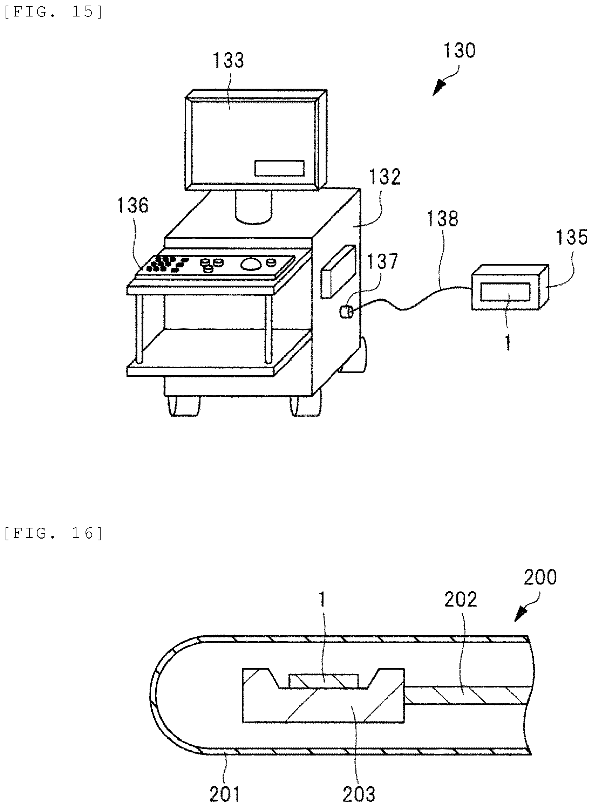

Hereinafter, the whole structure of the ultrasonic examination device of any one of the first to third embodiments will be described with reference to Fig. 15. Fig. 15 is a perspective view illustrating the ultrasonic examination device of the present embodiment.

The ultrasonic examination device of the present embodiment is a medical diagnostic device which uses permeability of sound waves, and can visualize an inner portion of a living body which cannot be seen from the outside, by imaging in real time by using ultrasonic waves exceeding an audible range. As shown in Fig. 15, the ultrasonic examination device (ultrasonic imaging device) 130 includes a main body 132, a display 133 disposed on an upper portion of the main body 132, an operation unit 136 installed on a front portion of the main body, and an ultrasonic probe 135 including a semiconductor chip 1. A cable (cord) 138 extends from the ultrasonic probe 135, and the cord is connected to the main body 132 at a connection portion 137. The operation unit 136 is an input device such as a track ball, a keyboard, or a mouse, or a combination thereof. The display 133 is a display device which displays an image-processed diagnostic image.

The semiconductor chip 1 corresponds to, for example, the semiconductor chip 1 shown in Fig. 1. That is, the capacitive type device 100 and the charging condenser 101 shown in Fig. 3 are mounted in the ultrasonic probe 135. In addition, the switch 102, the DC bias blocking circuit 107, the transception isolation circuit 108, and the amplifier circuit 109 shown in Fig. 3 are mounted in the ultrasonic probe 135, for example. The switch controller 105, the filter 110, and the analogue-digital converter 111 shown in Fig. 3 are mounted in the main body 132, for example. In addition, the image display device 112 shown in Fig. 3 corresponds to the display 133. As in the third embodiment, the switch 102 may be mounted in the semiconductor chip 1.

An alternating current voltage and a direct current voltage are applied to the semiconductor chip 1 from the transmission power source 104 and the DC bias power source 103 of the main body 132 via the cable 138. The ultrasonic probe 135 is a transmission and reception unit of ultrasonic waves. The semiconductor chip 1 is installed on a leading end surface of a probe case forming the ultrasonic probe 135 to have its main surface (a surface on which the plurality of oscillators are formed) face the outside.

During ultrasonic diagnosis, a leading end of the ultrasonic probe 135 is placed on a surface of an examinee (living body), and then moves for scanning while shifting from the position of the surface of the examinee where the leading end of the ultrasonic probe 135 is placed. When the ultrasonic probe 135 is a blood vessel catheter, the ultrasonic probe 135 is inserted into a blood vessel and scans therein. In this case, an ultrasonic pulse of a few MHz is transmitted to the inside of the examinee from the ultrasonic probe 135 which is made to approach a body surface or an inner wall of the blood vessel, and a reflected wave (echo) is received from a tissue boundary in which an acoustic impedance is different. Accordingly, a tomographic image of tissue of the living body displayed on the display 133 can be obtained, and information regarding an object to be diagnosed can be known. Distance information of a reflector can be obtained based on a time interval from the time that the ultrasonic wave is transmitted until the time that the ultrasonic wave is received. In addition, information on the presence or quality of the reflector can be obtained based on a level or an external form of the reflected wave.

In the present embodiment, the cell 2 of the ultrasonic transducer and the charging condenser 5 are formed in one semiconductor chip 1 as described in the first embodiment with reference to Figs. 1 to 3. Accordingly, when ultrasonic waves are transmitted and received by using the capacitive type device 100 shown in Fig. 3, a direct current voltage can be applied to the capacitive type device 100 from the charging condenser 101, which is charged in advance by using the DC bias power source 103, and the transmitting and receiving operation can be performed, although a direct current voltage is not applied to the semiconductor chip 1 from the DC bias power source 103. Accordingly, even when a short circuit breakdown occurs in the ultrasonic probe 135 shown in Fig. 15, and a current leaks into the living body from the semiconductor chip 1, a current can be prevented from directly flowing into the living body from the DC bias power source 103 shown in Fig. 3. That is, safety of the ultrasonic examination device can be enhanced.

In addition, a parasitic capacity generated in the voltage applying path can be greatly reduced by mix-mounting the cell 2 and the condenser 5 in one semiconductor chip 1, and accordingly, the cell 2 can be operated for a long time. Since the cell 2 and the condenser 5 are mix-mounted in one semiconductor chip 1, the ultrasonic probe 135 can be miniaturized. Therefore, performance of the ultrasonic examination device can be enhanced.

### (Fifth Embodiment)

Hereinafter, a structure of a probe which is an ultrasonic probe provided with the semiconductor chip of any one of the first to third embodiments, and is used in a blood vessel catheter will be described with reference to Fig. 16. Fig. 16 is a cross-sectional view illustrating the ultrasonic probe of the present embodiment. In Fig. 16, illustration of detailed cross-sectional structures of a probe assembly 203 and a wire 202 is omitted.

As shown in Fig. 16, the ultrasonic probe 200 of the present embodiment includes a catheter tube 201 which is a container insertable into a blood vessel of a living body. The wire 202 and the probe assembly 203 connected to a leading end of the wire 202 are arranged in the catheter tube 201. The semiconductor chip 1 described in any one of the first to third embodiments is fixed to a surface of the probe assembly 203 facing an inner wall of the catheter tube 201, which is a portion of the surface of the probe assembly 203.

That is, the semiconductor chip 1 includes the capacitive type device 100 and the charging condenser 101 shown in Fig. 3. The first electrode of the capacitive type device 100 and the third electrode of the charging condenser 101 are connected to the DC bias power source 103 via the switch 102, and the second electrode of the capacitive type device 100 and the fourth electrode of the charging condenser 101 are connected to the transmission power source 104, and furthermore, are connected to the image display device 112 via the amplifier 109, etc.

The wire 202 and the probe assembly 203 shown in Fig. 16 are not fixed to the catheter tube 201, and are movable in the catheter tube 201 in an extension direction of the catheter tube 201.

The ultrasonic probe 200 of the catheter type described above may be inserted into a blood vessel, for example, and can obtain an ultrasonic tomographic image by performing linear scanning or radial scanning. When linear scanning is performed by using the ultrasonic probe 200, the probe assembly 203 scans the extension direction in the catheter tube 201. In addition, when radial scanning is performed by using the ultrasonic probe 200, the probe assembly 203 is rotated about an axis of the extension direction in the catheter tube 201.

Since the probe assembly 203 and the semiconductor chip 1 are protected by the catheter tube 201, there is no possibility that the semiconductor chip 1 comes into contact with an examinee and a current leakage occurs. However, when the catheter tube 201 is damaged, a current may leak into the examinee from the semiconductor chip 1 through a blood vessel.

Herein, when the switch 102 and the charging condenser 101 shown in Fig. 3 are not installed, and ultrasonic waves are transmitted and received by using the capacitive type device 100, there is a need to always supply a direct current bias voltage to the capacitive type device 100 from the DC bias power source 103. In this case, when the current leakage occurs, there is a high possibility that a current directly and continuously flows into the examinee from the DC bias power source 103 via the semiconductor chip 1, and thus it is difficult to guarantee safety of the ultrasonic probe.

In contrast, in the present embodiment, the cell 2 of the ultrasonic transducer and the charging condenser 5 are formed in one semiconductor chip 1 as described in the first embodiment with reference to Figs. 1 to 3. Therefore, when ultrasonic waves are transmitted and received, a direct current voltage can be applied to the capacitive type device 100 from the charging condenser 101, which is charged in advance by using the DC bias power source 103, and the transmitting and receiving operation can be performed, although a direct current voltage is not applied to the semiconductor chip 1 from the DC bias power source 103. Accordingly, even when a short circuit breakdown occurs in the ultrasonic probe 200, and a current leaks into the examinee from the semiconductor chip 1, a current can be prevented from directly flowing into the living body from the DC bias power source 103 shown in Fig. 3. That is, safety of the ultrasonic probe can be enhanced.

In addition, a parasitic capacitance generated in the voltage applying path can be greatly reduced by mix-mounting the cell 2 and the condenser 5 in one semiconductor chip 1, and accordingly, the cell 2 can be operated for a long time. In addition, since the cell 2 and the condenser 5 are mix-mounted in one semiconductor chip 1, the ultrasonic probe 200 can be miniaturized. Therefore, performance of the ultrasonic probe can be enhanced.

### Reference Signs List

- 1A:: cell area
- 1B:: condenser area
- 2:: cell
- 5:: condenser
- 6, 7, 27:: upper electrode
- 8, 28:: lower electrode
- 9:: substrate
- 10, 55:: cavity
- 11:: material film
- 12:: membrane

## Claims

1. An ultrasonic examination device comprising:
a capacitive ultrasonic transducer which is formed of a first condenser (100) which comprises a first electrode (8), a cavity (10) on the first electrode (8), and a second electrode (6) formed on the cavity (10);
a second condenser (101) which comprises a third electrode (8) and a fourth electrode (7) above the third electrode (8), and is connected to the first condenser (100) in parallel;
**characterised by**
a switch element (102) which is connected between any one of the third electrode (8) or the fourth electrode (7), and a direct current power source (103)
a mechanism (113) for measuring a DC bias voltage of the first condenser (100); and
a mechanism (114) for adjusting a signal gain in accordance with the measured DC bias voltage,
wherein the first condenser (100) and the second condenser (101) are formed in a same semiconductor chip (1).

2. The ultrasonic examination device of claim 1, wherein the first condenser (100) and the second condenser (101) are arranged on a substrate (9) in a direction parallel to a main surface of the substrate (9).

3. The ultrasonic examination device of claim 2, wherein the first electrode (8) and the third electrode (8) are integrated into each other, and the second electrode (6) and the fourth electrode (7) are integrated into each other.

4. The ultrasonic examination device of claim 1, wherein the second condenser (101) has a low receive sensitivity of a sound wave, in comparison to the first condenser (100).

5. The ultrasonic examination device of claim 2, wherein a conductive film (11) which is spaced from both of the third electrode (8) and the fourth electrode (7) is formed between the third electrode (8) and the fourth electrode (7) on a position adjacent to the cavity (10).

6. The ultrasonic examination device of claim 1, wherein the switch element (102) is formed in the semiconductor chip (1).

7. The ultrasonic examination device of claim 1, wherein
the first condenser (100) is a variable capacitance element, and
the second condenser (101) is a fixed capacitance element.

8. The ultrasonic examination device of claim 1, wherein, when the switch element (102) is turned off, ultrasonic waves are transmitted and received using the capacitive ultrasonic transducer.

9. The ultrasonic examination device of claim 1, wherein the second condenser (101) is formed under the first condenser (100).

10. The ultrasonic examination device of claim 9, wherein a distance between the third electrode (8) and the fourth electrode (7) is smaller than a distance between the first electrode (8) and the second electrode (6).

11. An ultrasonic probe (135) comprising
the ultrasonic examination device of any of claims 1, 2 and 6-8, and
the semiconductor chip (1) including the first condenser (100) and the second condenser (101).

## Patentansprüche

1. Ultraschall-Untersuchungsvorrichtung, umfassend:
einen kapazitiven Ultraschallwandler, der aus einem ersten Kondensator (100) gebildet ist, der eine erste Elektrode (8), einen Hohlraum (10) auf der ersten Elektrode (8) und eine zweite Elektrode (6), die auf dem Hohlraum (10) gebildet ist, umfasst;
einen zweiten Kondensator (101), der eine dritte Elektrode (8) und eine vierte Elektrode (7) oberhalb der dritten Elektrode (8) umfasst und mit dem ersten Kondensator (100) parallelgeschaltet ist;
**gekennzeichnet durch**
ein Schaltelement (102), das zwischen der dritten Elektrode (8) und/oder der vierten Elektrode (7) und einer Gleichstromquelle (103) angeschlossen ist;
einen Mechanismus (113) zum Messen einer Gleichstromvorspannung des ersten Kondensators (100); und
einen Mechanismus (114) zum Einstellen einer Signalverstärkung gemäß der gemessenen Vorspannungsgleichspannung,
wobei der erste Kondensator (100) und der zweite Kondensator (101) in einem gleichen Halbleiterchip (1) ausgebildet sind.

2. Ultraschalluntersuchungsvorrichtung nach Anspruch 1, wobei der erste Kondensator (100) und der zweite Kondensator (101) auf einem Substrat (9) in einer Richtung parallel zu einer Hauptfläche des Substrats (9) angeordnet sind.

3. Ultraschalluntersuchungsvorrichtung nach Anspruch 2, wobei die erste Elektrode (8) und die dritte Elektrode (8) ineinander integriert sind, und die zweite Elektrode (6) und die vierte Elektrode (7) ineinander integriert sind.

4. Ultraschalluntersuchungsvorrichtung nach Anspruch 1, wobei der zweite Kondensator (101) im Vergleich zum ersten Kondensator (100) eine geringe Empfangsempfindlichkeit für Schallwellen aufweist.

5. Ultraschalluntersuchungsvorrichtung nach Anspruch 2, wobei ein leitender Film (11), der einen Abstand sowohl von der dritten Elektrode (8) als auch von der vierten Elektrode (7) hat, zwischen der dritten Elektrode (8) und der vierten Elektrode (7) an einer dem Hohlraum (10) benachbarten Position ausgebildet ist.

6. Ultraschalluntersuchungsvorrichtung nach Anspruch 1, wobei das Schaltelement (102) in dem Halbleiterchip (1) ausgebildet ist.

7. Ultraschalluntersuchungsvorrichtung nach Anspruch 1, wobei der erste Kondensator (100) ein Element mit variabler Kapazität ist, und der zweite Kondensator (101) ein Element mit fester Kapazität ist.

8. Ultraschalluntersuchungsvorrichtung nach Anspruch 1, wobei bei ausgeschaltetem Schaltelement (102) Ultraschallwellen unter Verwendung des kapazitiven Ultraschallwandlers gesendet und empfangen werden.

9. Ultraschalluntersuchungsvorrichtung nach Anspruch 1, wobei der zweite Kondensator (101) unter dem ersten Kondensator (100) ausgebildet ist.

10. Ultraschalluntersuchungsvorrichtung nach Anspruch 9, wobei ein Abstand zwischen der dritten Elektrode (8) und der vierten Elektrode (7) kleiner ist als ein Abstand zwischen der ersten Elektrode (8) und der zweiten Elektrode (6).

11. Ultraschallsonde (135) mit
der Ultraschalluntersuchungsvorrichtung nach einem der Ansprüche 1, 2 und 6-8, und
dem Halbleiterchip (1), der den ersten Kondensator (100) und den zweiten Kondensator (101) enthält.

## Revendications

1. Dispositif d'examen à ultrasons comprenant :
un transducteur ultrasonore capacitif qui est formé d'un premier condenseur (100) qui comprend une première électrode (8), une cavité (10) sur la première électrode (8) et une deuxième électrode (6) formée sur la cavité (10) ;
un second condenseur (101) qui comprend une troisième électrode (8) et une quatrième électrode (7) au-dessus de la troisième électrode (8), et est connecté au premier condenseur (100) en parallèle ;
**caractérisé par**
un élément de commutation (102) qui est connecté entre l'une quelconque de la troisième électrode (8) ou de la quatrième électrode (7), et une source d'alimentation en courant continu (103)
un mécanisme (113) pour mesurer une tension de polarisation continue du premier condenseur (100) ; et
un mécanisme (114) pour régler un gain de signal en fonction de la tension de polarisation continue mesurée,
dans lequel le premier condenseur (100) et le second condenseur (101) sont formés dans une même puce semi-conductrice (1).

2. Dispositif d'examen à ultrasons selon la revendication 1, dans lequel le premier condenseur (100) et le second condenseur (101) sont disposés sur un substrat (9) dans une direction parallèle à une surface principale du substrat (9).

3. Dispositif d'examen à ultrasons selon la revendication 2, dans lequel la première électrode (8) et la troisième électrode (8) sont intégrées l'une dans l'autre, et la deuxième électrode (6) et la quatrième électrode (7) sont intégrées l'une dans l'autre.

4. Dispositif d'examen à ultrasons selon la revendication 1, dans lequel le second condenseur (101) a une faible sensibilité de réception d'une onde sonore, par rapport au premier condenseur (100).

5. Dispositif d'examen à ultrasons selon la revendication 2, dans lequel un film conducteur (11) qui est espacé à la fois de la troisième électrode (8) et de la quatrième électrode (7) est formé entre la troisième électrode (8) et la quatrième électrode (7) sur une position adjacente à la cavité (10).

6. Dispositif d'examen à ultrasons selon la revendication 1, dans lequel l'élément de commutation (102) est formé dans la puce semi-conductrice (1).

7. Dispositif d'examen à ultrasons selon la revendication 1, dans lequel
le premier condenseur (100) est un élément à capacité variable, et
le deuxième condensateur (101) est un élément à capacité fixe.

8. Dispositif d'examen à ultrasons selon la revendication 1, dans lequel, lorsque l'élément de commutation (102) est éteint, des ondes ultrasonores sont transmises et reçues en utilisant le transducteur ultrasonore capacitif.

9. Dispositif d'examen à ultrasons selon la revendication 1, dans lequel le second condenseur (101) est formé sous le premier condenseur (100).

10. Dispositif d'examen à ultrasons selon la revendication 9, dans lequel une distance entre la troisième électrode (8) et la quatrième électrode (7) est inférieure à une distance entre la première électrode (8) et la deuxième électrode (6).

11. Sonde à ultrasons (135) comprenant
le dispositif d'examen à ultrasons selon l'une quelconque des revendications 1, 2 et 6 à 8, et
la puce semi-conductrice (1) comprenant le premier condenseur (100) et le second condenseur (101).
